# EUROPEAN PATENT APPLICATION

(11) **EP 3 456 840 A1**
(43) Date of publication of application: **20.03.2019**
(21) Application number: 17190994.8
(22) Date of filing: 14.09.2017
(51) Int. Cl.: C12Q 1/68, C12M 1/00, C12Q 1/24

(54) **METHOD OF ENRICHMENT OF MICRO-ORGANISMS IN WHOLE BLOOD**

(71) Applicant: Siemens Healthcare GmbH, 91052 Erlangen (DE)
(72) Inventor: MITRA, Nivedita, 560066 Bangalore (IN); VUTUKURU, Ramya, 560019 Bangalore, Karnataka (IN)
(74) Representative: Patentanwälte Bals & Vogel

(57) **Abstract**

A method (1) of enriching micro-organisms, in a metagenomics workflow, is disclosed. In an aspect of the invention, the method (1) includes obtaining said sample suspected to contain micro-organisms; concentrating said micro-organisms from said sample; and processing said micro-organisms to obtain genetic information specific to said micro-organisms. In another aspect of the invention, for concentrating said micro-organisms from said sample, the method (1) includes separating plasma from said whole blood sample; selectively removing white blood cells from said plasma; and isolating said micro-organisms from said plasma after selectively removing said white blood cells.

## Description

The present invention relates to a method of enrichment of micro-organisms in whole blood.

The metagenomics approach of nucleic acid sequencing is an unbiased way to analyze all the nucleic acid present in a sample. In comparison to pre-determined assay based methods of identification of pathogens, the metagenomics approach allows for identification of unsuspected micro-organisms. This enables further analysis of such pathogens which are not detected by traditional genome amplification approaches. The metagenomics approach also allows for the detection of antimicrobial resistance patterns that are distributed throughout the genome length. The presence of an overwhelming amount of human/host genomic deoxyribonucleic acid (DNA) background along with the target microbial DNA is the biggest technical challenge faced in the implementation of next generation sequencing workflows for identification of micro-organisms and their associated antibiotic resistance information from samples such as, but not limited to, blood, sputum, swab, and cerebrospinal fluid.

In a metagenomics study conducted on nasopharyngeal aspirates, disclosed in Yang et. al, J Clinical Microbiology, 49:3463-3469, it was revealed that up to 95% of raw next generation sequencing reads were that of human genomic DNA. In a sample like whole blood, this problem is bigger. The human genomic DNA present in the sample adds to sequencing time and cost. In addition, the subtraction of human genomic DNA component from that of the target-pathogen DNA is a computationally intensive process. There are multiple enrichment methods and kits available that can selectively eliminate human genomic DNA or selectively enrich pathogens. However, when such methods are used in a metagenomics workflow, adequate removal of human genomic DNA is not assured, thereby resulting in carry-over of significant amount of human genomic DNA. Such carry-over human genomic DNA can be a hindrance when very low-copies of pathogens are to be detected in the sample. If the workflow involves whole genomic amplification methods, there is a high vulnerability of amplification of nonspecific DNA. This makes the downstream sequencing and bioin-formatics lengthy and cumbersome.

In light of the above, there exists a need to provide a method that removes eukaryotic cells from a sample so as to make a more enriched pathogen sample available for the metagenomics workflow. There also exists a need to provide a method of removal of eukaryotic cells from a sample, which complements the current methods of DNA sequencing.

The object of the invention is therefore to provide a method for enrichment of micro-organisms in whole blood, thereby rendering an enriched pathogen sample for the metagenomics workflow of genomic sequencing.

The invention refers to a method of enrichment of micro-organisms in whole blood. The method includes obtaining a whole blood sample suspected to contain micro-organisms. Said sample may be obtained, for example, from a patient. The method further comprises concentrating said micro-organisms present in said sample. Concentrating micro-organisms enables removal of eukaryotic DNA background and efficient analysis of said micro-organisms. In a further step, the method comprises processing said micro-organisms to identify genetic information pertaining to said micro-organisms. Obtaining genetic information of said micro-organisms enables determining, for example, the antibiotic resistance profile of said micro-organisms.

In a preferred embodiment of the invention, in concentrating said micro-organisms from said sample, the method further comprises separating plasma from said whole blood. Whole blood comprises of red blood cells, white blood cells, platelets and blood plasma. On separation of blood plasma, most of the red blood cells and white blood cells are removed, thereby enabling depletion of human genomic DNA from the sample. Therefore, the plasma may contain micro-organisms and remaining white blood cells. An advantage of separation of blood plasma is that the eukaryotic DNA background is reduced significantly. Therefore, interferences in analysis of micro-organisms and cellular components of said micro-organisms are decreased. The method further includes selectively removing said remaining white blood cells from said plasma. The plasma is therefore left with micro-organisms after said selective removal of white blood cells. Advantageously, said selective removal of white blood cells does not affect said micro-organisms present in said sample. Therefore, said micro-organisms remain intact while further depletion of said eukaryotic cells is achieved. According to the invention, the method further includes isolating said micro-organisms from said plasma. On removal of said eukaryotic cells, said plasma contains micro-organisms which are isolated for further processing. Isolating said micro-organisms from said plasma enables obtaining a concentrated sample of said micro-organisms.

According to a preferred embodiment of the invention, said plasma is separated from said whole blood sample using centrifugation method. Said whole blood sample may be subjected to plasma fractionation. On centrifugation, red blood cells and other blood components settle at the bottom of the centrifugation tube, thereby forming a buffy coat as a top layer. Said separated plasma may be collected from above the layer of red blood cells. Centrifugation enables removal of most of the eukaryotic cells such as red blood cells as well as most of the white blood cells.

According to another preferred embodiment of the invention, said remaining white blood cells are selectively removed from said plasma by incubating said plasma with at least one matrix coated with a primary substrate. Said primary substrate may have affinity for white blood cells. Therefore, when said plasma containing white blood cells is brought in contact with said matrix coated with primary substrate, said white blood cells may bind to said primary substrate thereby forming a complex. Said complex may be separated from said plasma so as to remove white blood cells from said plasma. Therefore, further depletion of eukaryotic cells from said sample is achieved

According to a further embodiment of the invention, said method may further include selective removal of white blood cells in said plasma using at least one matrix coated with secondary substrate. Said secondary substrate may also have affinity for white blood cells. Said matrix coated with secondary substrate may be used advantageously to remove any white blood cells that may be present in said plasma after selective removal with a matrix coated with a primary substrate. When said plasma containing white blood cells is brought in contact with said matrix coated with secondary substrate, said white blood cells may bind to said secondary substrate thereby forming a complex. Said complex may be separated from said plasma so as to remove white blood cells from said plasma. Selectively removing white blood cells from said sample is a negative enrichment method. A negative enrichment method involves capturing and/or non-target cells such that target cells in the sample are intact and unaffected.

According to a preferred embodiment of the invention, said micro-organisms are isolated from said plasma by incubating said plasma with at least one matrix coated with a tertiary substrate. Isolation of micro-organisms from said sample is a positive enrichment method. A positive enrichment method involves isolation of target cells from a given sample, leaving behind the non-target cells and cellular components in the sample. Said tertiary substrate may have affinity for said micro-organisms. When said plasma containing micro-organisms is brought in contact with said matrix coated with tertiary substrate, said micro-organisms may bind to said tertiary substrate thereby forming a complex. Said complex may be separated from said plasma so as to isolate said micro-organisms from said plasma. Therefore, on isolation of said micro-organisms using said tertiary substrate, a concentrated sample of micro-organisms is obtained. Thus, maximum depletion of eukaryotic cellular background is achieved.

According to another preferred embodiment of the invention, said whole blood sample is centrifuged at a relative centrifugal force (RCF) ranging between 50 x g and 500 x g, and particularly between 100 x g and 300 x g. Centrifuging said whole blood sample at this RCF range advantageously allows for least human genomic DNA carry-over in said separated plasma.

According to an embodiment of the invention, said matrix for selective removal of white blood cells and for isolation of micro-organisms from said plasma may be chosen from a group that includes polystyrene beads and magnetic beads. Said magnetic bead comprises at least one particle of ferromagnetic, ferrimagnetic, super-magnetic or paramagnetic material. The surface of said matrix is adsorptive and is coated with at least one substrate. Said substrate may have affinity for either white blood cells or for micro-organisms. Spherical beads provide a greater surface area for binding of the desired entity to said coated substrate.

According to an embodiment of the invention, said primary substrate and said secondary substrate are chosen from a group comprising CD45, CD15, CD14, CD3, CD4, CD8 and CD19 proteins. CD proteins are used as markers for immunophenotyping, thereby enabling cells to be differentiated based on what molecules are present on their surface. Said CD proteins have a high affinity for white blood cells. Therefore, when a white blood cell in plasma is brought in contact with a matrix coated with a CD protein, a complex is formed between said white blood cell and said CD protein. Formation of complex enables efficient separation of such eukaryotic cells from said plasma.

According to an embodiment of the invention, said primary substrate and said secondary substrate are different. For example, if said primary substrate is a CD15 protein, said secondary substrate may be a CD45 protein.

According to an embodiment of the invention, said tertiary substrate is a pathogen binding protein and may be, for example, Apolipoprotein H, histone proteins, methyl-CpG-binding domains (MBD Proteins) or immune proteins. Said pathogen binding proteins have a high affinity for micro-organisms. Therefore, when a matrix coated with said tertiary substrate is brought in contact with said sample, said micro-organisms bind to said substrate and for a complex. Use of a tertiary substrate allows for microbial isolation from said sample, thereby ensuring removal of complete eukaryotic DNA background.

According to another embodiment of the invention, said white blood cells bound to said matrix are separated from said plasma using a magnet. If the matrix onto which said substrates are coated is a magnetic bead, the magnet attracts said magnetic bead, thereby separating the complex from the plasma easily.

According to yet another embodiment of the invention, said white blood cells bound to said matrix are separated from the plasma using centrifugation. If the matrix onto which said substrates are coated is a polystyrene bead, the complex formed can be separated from the plasma using centrifugation. On centrifugation of said sample, said polystyrene beads along with said bound eukaryotic cells form a pellet at the bottom of the centrifuge tube. As said micro-organisms remain in the supernatant formed as a result of centrifugation, elimination of said white blood cells from said sample is achieved.

The present invention also provides for a kit for carrying out the abovementioned method. According to the invention, said kit includes one or more matrices coated with a primary substrate and a secondary substrate for removal of eukaryotic cells from said sample. Said kit further includes one or more matrices coated with a tertiary substrate for isolation of micro-organisms from said sample, which can be used for the enrichment of micro organisms present in a sample.

The present invention is further described hereinafter with reference to illustrated embodiments shown in the accompanying drawings, in which:
- FIG 1: illustrates a schematic diagram of a flow chart of an embodiment of a method according to the invention.
- FIG 2: illustrates a graph depicting the effect of centrifugal speed on the human genomic DNA carry-over in the plasma.
- FIG 3: illustrates a graph depicting the change in the amounts of human genomic DNA to microbial DNA with the employment of enrichment steps according to the invention.

Hereinafter, embodiments for carrying out the present invention are described in detail. The various embodiments are described with reference to the drawings, wherein like reference numerals are used to refer to like elements throughout. In the following description, for purpose of explanation, numerous specific details are set forth in order to provide a thorough understanding of one or more embodiments. It may be evident that such embodiments may be practiced without these specific details.

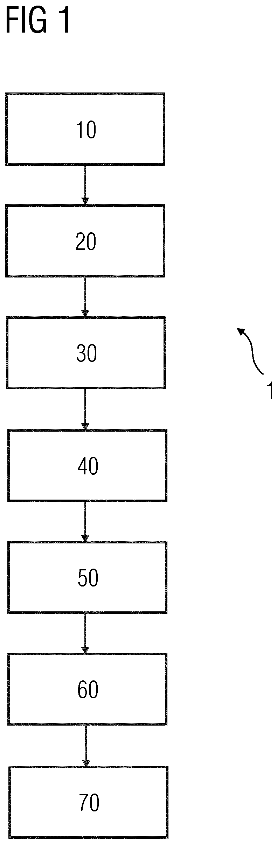

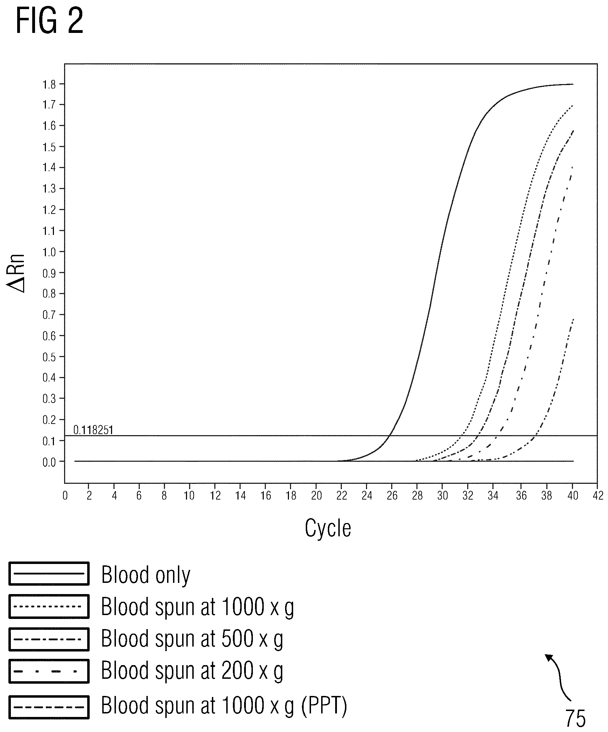

FIG 1 illustrates a schematic representation of a flowchart of a method 1 for enrichment of micro-organisms. The sample used in the present embodiment is a whole blood sample suspected to contain micro-organisms. Alternatively, other fluids of the human body that are known to be used for such analyses can also be used. The sample volume may be in the range between 8 milliliter and 15 milliliter. In the present embodiment, a sample volume of 8 milliliter is considered for enrichment. In the first step 10 of said method 1, said whole blood sample suspected to contain micro-organisms is subject to plasma fractionation. Said plasma is separated from said whole blood using centrifugation. 8 milliliter of whole blood sample is centrifuged at a relative centrifugal force (RCF) ranging between 50 x g and 500 x g, and more preferably between 150 x g and 250 x g for a time period in the range between 10 minutes to 30 minutes and preferably between 15 minutes to 20 minutes. The relative centrifugal force used for separation of plasma is approximately one fifth of the recommended usual speed for plasma fractionation. At higher speeds, the quantity of human genomic DNA observed in the plasma is higher. This may be because of shearing of eukaryotic cells at higher speeds, resulting in release of human genomic DNA into the plasma. A graph 75 depicting the effect of centrifugal speed on the human genomic DNA carry-over in the plasma is illustrated in FIG 2. The X-axis of the graph 75 represents the number of cycles of polymerase chain reaction for amplification of DNA present in the sample. The Y-axis of the graph 75 represents the amount of fluorescence produced and detected as a result of amplification of nucleic acids. The amount of fluorescence generated is directly proportional to the amount of DNA present in the sample being amplified. Therefore, as the polymerase chain reaction progresses, the amount of nucleic acids in the sample increases. If the amount of nucleic acid present in the sample is low, more number of cycles of polymerase chain reaction would be required to produce sufficient amount of amplified DNA. Therefore, greater number of amplification cycles would be required for generation of fluorescence beyond a certain threshold. In the present embodiment, said threshold of fluorescence is 0.118251. According to the graph 75, when a 50 micro liter sample containing only blood is amplified, the number of amplification cycles required to produce fluorescence above the threshold value is 25.62. 8 milliliter whole blood sample is spun at an RCF value of 1000 x g to obtain plasma. A 50 micro liter representative sample of said obtained plasma is used for a quantitative DNA analysis. Said 50 micro liter plasma sample is subjected to amplification. The number of amplification cycles required to produce fluorescence above said threshold value is 31.2. Further, an 8 milliliter whole blood sample is spun at an RCF value of 500 x g to obtain plasma. When a representative sample amount of 50 micro liter of said obtained plasma is subjected to amplification, the number of amplification cycles required to produce fluorescence above the threshold value is 32.46. Similarly, an 8 milliliter whole blood sample is spun at an RCF value of 200 x g to obtain plasma. When a representative sample amount of 50 micro liter of said obtained plasma is amplified, fluorescence is generated after 34.04 amplification cycles. The increase in the number of amplification cycles with reduction in the centrifugal speed is due to reduction in the amount of human genomic DNA in the plasma sample. At a higher centrifugal speed, cell shearing may occur, thereby allowing spilling of human genomic DNA from the blood cells into the sample. Therefore, spinning the sample at lower speeds reduces the risk of cell shearing and therefore reduces the amount of human genomic DNA in the sample.

At centrifugal speed of 200 x g, a buffy coat is formed in the centrifuge tube, and plasma is collected from the top layer. Centrifugation of said whole blood sample at said range of relative centrifugal force enables ∼1000 fold depletion of human genomic DNA from eukaryotic cells such as red blood cells, platelets, and white blood cells. In the second step 20 of said method 1, said separated plasma is treated further such that any white blood cells remaining in said plasma are removed. Therefore, at step 20, said plasma is incubated with at least one matrix coated with a primary substrate. In the present embodiment, said at least one matrix is a magnetic bead. Said primary substrate to be coated over said magnetic bead is chosen from a group of substrates that have affinity for white blood cells. In the present embodiment, said primary substrate is a CD45 protein. Said plasma is incubated with said at least one matrix for a time period ranging between 1 minute to 60 minutes and preferably between 10 minutes to 30 minutes. On incubation of said plasma with said CD45 proteins, said white blood cells bind to said CD45 proteins to form a complex. CD45 proteins selectively bind to any white blood cells that are present in said plasma. Once said complex is formed, at step 30, said matrix is removed using a magnet. Magnetic beads are attracted to a magnet, thereby enabling easy removal of said complex. In an embodiment, said plasma is further incubated with at least one matrix coated with a secondary substrate. Such secondary substrate is chosen from a group of substrates having affinity for white blood cells. In the present embodiment, said secondary substrate is CD15 protein. CD15 proteins selectively bind to any white blood cells that are remaining in said plasma, thereby forming a complex. Once said complex is formed, said matrix is removed using a magnet. At the end of the second step, ∼10,000 fold removal of human genomic DNA is achieved. This corresponds to removal of 99.99% of human genomic DNA.

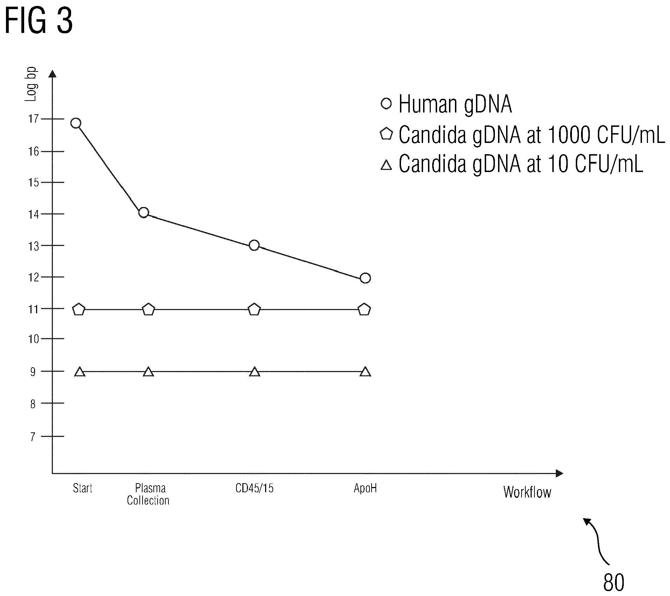

At step 40, said plasma is incubated with at least one matrix coated that is coated with a tertiary substrate. In the present embodiment, said matrix is a magnetic bead. Such tertiary substrate is chosen from a group of substrates that have an affinity for microbial cells. In the present embodiment, said tertiary substrate is Apolipoprotein H (ApoH) from ApoH Technologies®. ApoH proteins are plasmatic proteins that are capable of binding to micro-organisms that may be present in a given sample. Said plasma is incubated with said at least one matrix for a time period ranging between 1 minute to 60 minutes and preferably between 10 minutes to 30 minutes. On incubation of said plasma with said ApoH proteins, said micro-organisms bind to said ApoH proteins to form a complex. Once said complex is formed, at step 50, said matrix is removed using a magnet. Magnetic beads are attracted to a magnet, thereby enabling easy removal of said complex. This positive enrichment method for isolating said micro-organisms enables complete removal of human genomic DNA, thereby resulting in 10⁴ human genomic DNA base pairs per base pair of microbial DNA. A reduction in the amount of human genomic DNA to microbial DNA at each step of the method is illustrated as a graphical representation 80 in FIG 3. A 100,000 fold depletion of human genomic DNA is achieved through the abovementioned method steps. The loss of microbial cells is minimal in comparison to depletion of eukaryotic cells. At step 60, said micro-organisms can be separated from said complex for further downstream processing at step 70, such as lysis of microbial cells for isolation of microbial DNA. Said downstream processing may also include amplification of the genomic DNA of said micro-organisms and DNA sequencing.

The instant teachings also provide kits designed to expedite performance of the subject methods. Kits serve to expedite the performance of the methods of interest by assembling two or more components required for carrying out the disclosed methods. Kits may contain components in pre-measured unit amounts to minimize the need for measurements by end-users. Kits may include instructions for performing one or more of the disclosed methods. Preferably, said kit components are optimized to operate in conjunction with one another.

In an embodiment, kits comprise primary substrate and secondary substrate for removal of eukaryotic cells/white blood cells from said sample. Said kit further includes tertiary substrate for isolation of micro-organisms from said sample. Said primary and secondary substrates are chosen from a group comprising CD45, CD15, CD4, CD3, and CD8. Said tertiary protein is a pathogen binding protein. Said matrix is a magnetic bead. Alternatively, said matrix may be a polystyrene bead. In an embodiment, said primary substrate coated onto one or more matrices is different from said secondary substrate. Said primary and secondary substrates enable removal of white blood cells from said sample, thereby leaving behind micro-organisms unaffected. Said tertiary substrate is then used to isolate said unaffected micro-organisms to obtain an enriched and concentrated sample of micro-organisms for further downstream processing.

The abovementioned method of enrichment of micro-organisms involves a combination of negative and positive enrichment methods. Negative enrichment ensures elimination of non-target cells from the sample. Therefore, the non-target background is removed thereby providing a concentrated sample containing target cells. When followed by a positive enrichment step, the target cells are separated from any non-target cells that may have been left out in the process of negative enrichment. Therefore, the combination of negative and positive enrichment steps yields a highly enriched and concentrated sample of target cells, which are micro-organisms, in the present embodiment. The abovementioned method of enrichment of micro-organisms can be integrated efficiently with the downstream sample preparation methods. Library preparation steps such as dA tailing, adapter ligation is more efficient due to significant reduction in background human genomic DNA. The method also enables efficient amplification of whole genome of micro-organisms. A better ratio of microbial to human DNA sequences read allows for reduction in the time for genome sequencing. Thus, this enrichment method can be integrated with the sample preparation methods of any gene sequencing workflow that is used for metagenomics analysis of pathogens in a complex sample like whole blood.

The foregoing examples have been provided merely for the purpose of explanation and are in no way to be construed as limiting of the present invention disclosed herein. While the invention has been described with reference to various embodiments, it is understood that the words, which have been used herein, are words of description and illustration, rather than words of limitation. Further, although the invention has been described herein with reference to particular means, materials, and embodiments, the invention is not intended to be limited to the particulars disclosed herein; rather, the invention extends to all functionally equivalent structures, methods and uses, such as are within the scope of the appended claims. Those skilled in the art, having the benefit of the teachings of this specification, may effect numerous modifications thereto and changes may be made without departing from the scope and spirit of the invention in its aspects.

### List of reference numbers

- 1: Method of enrichment of micro-organisms in a metagenomics workflow
- 75: Graph depicting depicting the effect of centrifugal speed on the human genomic DNA carry-over in the plasma
- 80: Graph depicting reduction in the amount of human genomic DNA to microbial DNA at each step of the method

## Claims

1. A method (1) of enrichment of micro-organisms, in a metagenomics nucleic acid sequencing workflow, from a whole blood sample suspected to contain micro-organisms, said method (1) comprising:
a. obtaining said whole blood sample suspected to contain micro-organisms;
b. concentrating said micro-organisms from said sample; and
c. processing said micro-organisms to obtain genetic information specific to said micro-organisms;
**characterized in that** in concentrating said micro-organisms, said method further comprises:
d. separating plasma from said whole blood sample;
e. selectively removing white blood cells from said plasma; and
f. isolating said micro-organisms from said plasma after selectively removing said white blood cells.

2. The method (1) according to claim 1, wherein the method step d of separating said plasma from said whole blood sample comprises the usage of centrifugation method.

3. The method (1) according to claim 2, wherein said whole blood sample is centrifuged at a relative centrifugal force (RCF) ranging between 50 x g and 500 x g.

4. The method (1) according to claim 1, wherein selectively removing white blood cells from said plasma comprises incubating said plasma with at least one matrix coated with a primary substrate having affinity for white blood cells.

5. The method (1) according to claim 4, wherein the method step f of selectively removing said white blood cells from said plasma comprises incubating said plasma with at least one matrix coated with a secondary substrate having affinity for white blood cells, after usage of said primary substrate.

6. The method (1) according to claim 4 and 5, wherein said primary substrate and said secondary substrate and chosen from a group comprising of CD45, CD15, CD4, CD3, and CD8.

7. The method (1) according to claim 6, wherein said primary and secondary substrates are different from each other.

8. The method (1) according to claim 1, wherein the method step f of isolating said micro-organisms from said plasma comprises incubating said plasma with at least one matrix coated with a tertiary substrate having affinity for said micro-organisms.

9. The method (1) according to claim 8, wherein said tertiary substrate is a pathogen binding protein.

10. The method (1) according to claim 4, 5 and 7 wherein said matrix is chosen from a group comprising of polystyrene beads and magnetic beads.

11. The method (1) according to claim 4, 5 and 8, wherein said white blood cells bound to said matrix are removed from said plasma using a magnet.

12. The method (1) according to claim 4, 5 and 8, wherein said white blood cells bound to said matrix are removed from said plasma using centrifugation.

13. A kit for carrying out a method (1) of enrichment of micro-organisms in a metagenomics workflow, said kit comprising:
a. one or more matrices coated with a primary substrate for removal of white blood cells, wherein said primary substrate has an affinity for white blood cells;
b. one or matrices coated with a secondary substrate for removal of white blood cells, wherein said secondary substrate has an affinity for white blood cells; and
c. one or more matrices coated with a tertiary substrate for isolation of micro-organisms, wherein said tertiary substrate has an affinity for micro organisms.

14. The kit according to claim 13, wherein said primary substrate and secondary substrate are chosen from a group comprising of CD45, CD15, CD4, CD3, and CD8.

15. The kit according to claim 13, wherein said tertiary substrate is a pathogen binding protein.

16. The kit according to claim 13, wherein said matrix is chosen from a group comprising of polystyrene beads and magnetic beads.

17. The use of kit as claimed in claims 13-16 to carry out a method (1) of enrichment of micro-organisms in a metagenomics workflow as claimed in claim 1-12.
